Europäisches Patentamt

European Patent Office

Office européen des brevets

(11) Publication number: 0 523 294 A1

# EUROPEAN PATENT APPLICATION

(21) Application number: 91306435.8

(22) Date of filing: 16.07.91

(51) Int. Cl.⁵: A61K 7/42, A61K 7/48, A61K 47/02

(43) Date of publication of application:
20.01.93 Bulletin 93/03

(84) Designated Contracting States:
DE FR GB

(71) Applicant: TAYCA CORPORATION
3-47, Funa-machi 1-chome, Taisho-ku
Osaka-shi, Osaka-fu(JP)

(72) Inventor: Maki, Tsuyoshi
960-109, Nakano Saidaiji
Okayama-shi, Okayama-ken(JP)
Inventor: Idei, Shunji
328, Ikoshi Saidaiji
Okayama-shi, Okayama-ken(JP)

(74) Representative: Myerscough, Philip Boyd et al
J.A. Kemp & Co. 14 South Square, Gray's Inn
London WC1R 5LX(GB)

(54) Paraffin hydrocarbon dispersion of titanium dioxide fine-particles.

(57) A paraffin hydrocarbon dispersion of titanium dioxide fine-particles having excellent dispersion stability comprises specified proportions of titanium dioxide fine particles, silica fine particles, dispersing agent and paraffin hydrocarbon and can be widely used for cosmetics providing an ultraviolet ray shielding effect.

The present invention relates to a paraffin hydrocarbon dispersion of titanium dioxide fine-particles, and in particular, to a paraffin hydrocarbon dispersion of titanium dioxide fine-particles for use in cosmetics.

Titanium dioxide fine-particles have been used for various anti-sunburn cosmetics, since such titanium dioxide particles show high transparency and high ultraviolet ray absorption and are harmless. Thus titanium dioxide fine-particles can be used in such compositions as anti-sunburn cream, lotion, milky lotion and ointment, are safe to use on the skin and exhibit an ultraviolet ray shielding effect.

Such cosmetics have been prepared by dispersing the titanium dioxide fine-particles in oily substances such as liquid paraffin, olive oil, petrolatum or squalane, and then, if necessary, adding a surface active agent, a perfumery, a coloring agent, a nutritious agent and the like. For example, JP-A-49-450 discloses in the Example that a paraffin hydrocarbon dispersion of titanium dioxide fine-particles is used as a raw material of an anti-sunburn cream.

When an oil dispersion of titanium dioxide fine-particles is used to prepare cosmetics, the problem of dust generation during handling of titanium dioxide fine-particles is solved and further the time for dispersing the titanium dioxide fine-particles can be saved.

When a paraffin hydrocarbon such as squalane is used as an oil component, it is found that there is a problem with dispersion stability, namely titanium dioxide fine-particles sediment in a relatively short time even when a dispersing agent is used.

We have found that by adding silica fine-particles, a paraffin hydrocarbon dispersion of titanium dioxide fine-particles is obtained having excellent dispersion stability.

The present invention provides a paraffin hydrocarbon dispersion of titanium dioxide fine-particles comprising 10 - 50 weight % of titanium dioxide fine-particles, 0.5 - 4 weight % of silica fine-particles, 0.3 - 4 weight % of dispersing agent and 42 - 89.2 weight % of paraffin hydrocarbon.

The titanium dioxide fine-particles used in the dispersion according to the present invention suitably have an average particle diameter of 0.01 to 0.1 $\mu$m. The crystal structure of the particle is not restricted to any particular one, however, rutile-type ones are preferable. Also, titanium dioxide fine-particles coated with an oxide or salt of a metal such as Al, Si, Zr or Fe can be used.

In particular, titanium dioxide fine-particles coated with a metal salt of a higher fatty acid, for example aluminum stearate or aluminum laurate, are preferred, since they are dispersed easily in paraffin hydrocarbon. The coating amount of the metal oxide or the metal salt of higher fatty acid is ordinarily in the range of 5 - 20 weight % based on the titanium dioxide fine-particles.

As the silica fine-particles, particles having a BET specific surface area of 100 - 300 $m^2$/g can be used.

As the dispersing agents, an organic dispersing agent which can be solved in paraffin hydrocarbon are usable. In particular, derivatives of dicarboxylic acids having at least one hydroxyl group are preferred. As such dispersing agent, esters of an oxy-acid such as glycolic acid, lactic acid, malic acid, tartaric acid and citric acid, and a higher alcohol such as capryl alcohol, lauryl alcohol, myristyl alcohol, palmityl alcohol and stearyl alcohol, may be exemplified. In particular, diesters of malic acid and iso-stearyl alcohol are most preferable.

As a paraffin hydrocarbon, squalane and liquid paraffin may be used. In particular, olive squalane is preferable. In general, a paraffin hydrocarbon is better on odor and acidification relative to animal oil and vegetable oil, so that the quality is stable for a long period.

The paraffin hydrocarbon dispersion of titanium dioxide fine-particles according to the present invention is obtained by blending the following constituents.

titanium dioxide fine-particles: 10 - 50 weight %

silica fine-particles: 0.5 - 4 weight % preferably 1.0 - 3.0 weight %

dispersing agent: 0.3 - 4 weight % preferably 0.5 - 3.0 weight %

paraffin hydrocarbon: 42 - 89.2 weight %

When the blending ratio of titanium dioxide fine-particles is less than 10 weight %, a dispersion stability of titanium dioxide fine-particles tends to lower. When the blending ratio is more than 50 weight %, the titanium dioxide fine-particles or the obtained dispersion are apt to change with the passage of time during the dispersion is transferred and stored. When the blending ratio of silica fine-particles is less than 0.5 weight %, the improvement of dispersion stability is not sufficient. When the blending ratio is more than 4 weight %, the high dispersion stability can not be expected with regard to the amount used, deteriorating its economy. When the blending ratio of the dispersing agent is less than 0.3 weight %, a dispersion degree of the titanium dioxide fine-particles is not sufficiently high. On the contrary, in case that it is more than 4 weight %, its disperse stability is deteriorated.

First, a dispersing agent is added to paraffin hydrocarbon and they are blended, titanium dioxide fine-particles and silica fine-particles are added to the resultant mixture, thereby obtaining a pre-mixed dispersion.

Next, the thus-obtained pre-mixed dispersion is completely dispersed by a dispersing machine, thereby obtaining a uniform dispersion according to the present invention. Ordinal type of the dispersing machines can be used. For example, a sand mill filled with zircon beads is preferably used so as to pulverize titanium dioxide fine-particles and silica fine-particles and to disperse them.

The paraffin hydrocarbon dispersion of titanium dioxide fine-particles according to the present invention has not more than 15 mm, preferably not more than 10 mm of a depth of the supernatant liquid of the dispersion after allowing to stand at a temperature of 40°C for 90 days.

The paraffin hydrocarbon dispersion of titanium dioxide fine-particles according to the present invention has a good dispersion stability, so it is possible to widely use in cosmetics necessitating ultraviolet ray shielding effect.

Examples 1 - 12 and Comparative Examples 1 - 6

Respective components shown in Table 1 were mixed with these ratios shown to produce a paraffin hydrocarbon dispersion of titanium dioxide fine-particles. The dispersion was tested in its disperse stability. The results of the test is shown in Table 1.

(Components)

Titanium dioxide fine-particles: particles having an avarage particle diameter of 0.02 $\mu$m and covered with aluminum stearate of 10 weight % based on titanium dioxide fine-particles.

Silica fine-particles: particles having a BET specific surface area of 220 $m^2/g$.

Dispersing agent: diester of malic acid and isostearyl alcohol.

Paraffin hydrocarbon: olive squalane.

(Producing process)

First, a paraffin hydrocarbon and a dispersing agent were charged and blended in a mixing tank, titanium dioxide fine-particles and silica fine-particles (Examples only) were added stirring and blending, thereby obtaining a pre-mixed dispersion.

Next, the thus-obtained pre-mixed dispersion was supplied to a sand mill and the titanium dioxide fine-particles and silica fine-particles (Examples only) were pulverized and dispersed. An open-type sand mill which was filled with zircon beads of an average diameter of 0.8 mm at a void ratio of 50% was used.

(Dispersion stability test)

200 $cm^3$ of the thus-obtained dispersion were placed in a measuring cylinder and allowed to stand in an artificial incubator of 40°C for 90 days. After that, the depth of the supernatant liquid was measured, which is a value of the dispersion stability. The shallower the depth of the supernatant liquid is, the better the dispersion stability.

Table 1

| Examples & Comparative Examples | | composition ratio (weight %) | | | | depth of supernatant liquid (mm) |
|---|---|---|---|---|---|---|
| | | titanium dioxide fine-particle | silica fine-particle | dispersing agent | paraffin hydrocarbon | |
| Comparison | 1 | 30 | 0 | 0.3 | 69.7 | 20 |
| Example | 1 | 30 | 1.0 | 0.3 | 68.7 | 10 |
| Example | 2 | 30 | 2.0 | 0.3 | 67.7 | 12 |
| Comparison | 2 | 30 | 0 | 0.9 | 69.1 | 25 |
| Example | 3 | 30 | 1.0 | 0.9 | 68.1 | 0 |
| Example | 4 | 30 | 2.0 | 0.9 | 67.1 | 2 |
| Comparison | 3 | 30 | 0 | 1.5 | 68.5 | 32 |
| Example | 5 | 30 | 1.0 | 1.5 | 67.5 | 2 |
| Example | 6 | 30 | 2.0 | 1.5 | 66.5 | 10 |
| Comparison | 4 | 30 | 0 | 3.0 | 67.0 | 40 |
| Example | 7 | 30 | 1.0 | 3.0 | 66.0 | 9 |
| Example | 8 | 30 | 2.0 | 3.0 | 65.0 | 11 |
| Comparison | 5 | 50 | 0 | 1.0 | 49.0 | 29 |
| Example | 9 | 50 | 1.5 | 1.0 | 47.5 | 0 |
| Example | 10 | 50 | 3.0 | 1.0 | 46.0 | 2 |
| Comparison | 6 | 10 | 0 | 0.9 | 89.1 | 32 |
| Example | 11 | 10 | 1.0 | 0.9 | 88.1 | 8 |
| Example | 12 | 10 | 2.0 | 0.9 | 87.1 | 3 |

Claims

1. A paraffin hydrocarbon dispersion of titanium dioxide fine-particles, comprising 10 - 50 weight % of titanium dioxide fine-particles, 0.5 - 4 weight % of silica fine-particles, 0.3 - 4 weight % of dispersing agent and 42 - 89.2 weight % of paraffin hydrocarbon.

2. A dispersion according to claim 1, wherein said silica fine-particles have a BET specific surface area of 100-300 $m^2$/g.

3. A dispersion according to claim 1 or 2, wherein said paraffin hydrocarbon is squalane or liquid paraffin.

4

4. A dispersion according to claim 3 wherein said paraffin hydrocarbon is olive squalane.

5. A dispersion according to any one of the preceding claims wherein said dispersing agent is a derivative of a dicarboxylic acid having at least one hydroxyl group.

6. A dispersion according to claim 5 wherein the dispersing agent is an ester of an oxy-acid and a higher alcohol.

7. A dispersion according to any one of the preceding claims wherein the titanium dioxide fine-particles are of the rutile type.

8. A dispersion according to any one of the preceding claims, wherein said titanium dioxide fine-particles have an average particle diameter of 0.01 - 0.1 $\mu$m.

9. A dispersion according to any one of the preceding claims, wherein said titanium dioxide fine-particles are coated with a metal oxide or a metal salt of a higher fatty acid.

10. A cosmetic preparation comprising a paraffin hydrocarbon dispersion as claimed in any one of the preceding claims.

European Patent
Office

# EUROPEAN SEARCH REPORT

Application Number

EP    91 30 6435

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 400 546 (HELENE CURTIS , INC.)<br>* page 5, line 8 - line 11; claims 1-36 *<br>--- | 1-10 | A61K7/42<br>A61K7/48<br>A61K47/02 |
| A | FR-A-2 622 440 (L'OREAL)<br>* page 8, line 1 - line 26 *<br>* page 9, line 26 - page 10, line 16 *<br>* page 10, line 31 - page 11, line 2; claims 1-13 *<br>--- | 1-10 | |
| A | FR-A-2 248 023 (HENKEL & CIE G.M.B.H.)<br>* page 2, line 15 - line 20 *<br>* page 4; example 2 *<br>* page 5; examples 6,9 *<br>* claims 1-6 *<br>--- | 1-10 | |
| A | GB-A-2 205 088 (TIOXIDE GROUP PLC)<br>* page 8, line 3 - page 10, line 20; claims 1-22 *<br>--- | 1-10 | |
| A | FR-A-2 646 346 (JEAN NOEL THOREL)<br>* the whole document *<br>--- | 1-10 | TECHNICAL FIELDS SEARCHED (Int. Cl.5 ) |
| A | PATENT ABSTRACTS OF JAPAN<br>vol. 7, no. 131 (C-169)(1276) 8 June 1983<br>& JP-A-58 043 912 ( SHISEIDO K.K. ) 14 March 1983<br>* abstract *<br>--- | 1-10 | A61K |
| A | GB-A-1 372 701 (JOHNSON & JOHNSON)<br>* the whole document *<br>----- | 1-10 | |

The present search report has been drawn up for all claims

| Place of search | Date of completion of the search | Examiner |
|---|---|---|
| BERLIN | 18 FEBRUARY 1992 | SIATOU E. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
........................................................................................
& : member of the same patent family, corresponding
    document

EPO FORM 1503 03.82 (P0401)